# EUROPEAN PATENT SPECIFICATION

(11) **EP 0 981 650 B1**
(45) Date of publication and mention of the grant of the patent: **13.12.2006**
(21) Application number: 98924658.2
(22) Date of filing: 13.05.1998
(51) Int. Cl.: C12Q 1/68

(54) **MOLECULAR DETECTION OF CHROMOSOME ABERRATIONS**
MOLEKULARER NACHWEIS VON CHROMOSOMALEN VERÄNDERUNGEN
DETECTION MOLECULAIRE D'ANOMALIES CHROMOSOMIQUES

(30) Priority: 13.05.1997 EP 97201440
(43) Date of publication of application: 01.03.2000
(73) Proprietor: Erasmus Universiteit Rotterdam, 3015 GE Rotterdam (NL)
(72) Inventor: VAN DONGEN, Jacobus, Johannus, Maria, NL-2914 LE Nieuwerkerk aan de Ijssel (NL); LANGERAK, Anthonie, Willem, NL-2992 WJ Barendrecht (NL)
(74) Representative: Grünecker, Kinkeldey, Stockmair & Schwanhäusser Anwaltssozietät
(86) International application number: PCT/NL1998/000270
(87) International publication number: WO 1998/051817

(56) References cited:
- EP-A- 0 430 402
- EP-A- 0 500 290
- WO-A-96/17958
- US-A- 5 538 869
- TKACHUK ET AL.: "Detection of bcr-abl fusion in chronic myelogeneous leukemia by in situ hybridization" SCIENCE, vol. 250, 1990, pages 559-562, XP002044140
- TKACHUK D C ET AL: "CLINICAL APPLICATIONS OF FLUORESCENCE IN SITU HYBRIDIZATION" GENETIC ANALYSIS TECHNIQUES AND APPLICATIONS, vol. 8, no. 2, 1 April 1991, pages 67-74, XP000262526
- THOMPSON C T ET AL: "CYTOGENETIC PROFILING USING FLUORESCENCE IN SITU HYBRIDIZATION (FISH) AND COMPARATIVE GENOMIC HYBRIDIZATION (CGH)" JOURNAL OF CELLULAR BIOCHEMISTRY. SUPPLEMENT, no. SUPPL. 17G, 1993, pages 139-143, XP000612761
- MANOIR DU S ET AL: "DETECTION OF COMPLETE AND PARTIAL CHROMOSOME GAINS AND LOSSES BY COMPARATIVE GENOMIC IN SITU HYBRIDIZATION" HUMAN GENETICS, vol. 90, 1993, pages 590-610, XP000606241
- LEUKEMIA vol. 9, 1995, pages 762 - 769

## Description

The invention relates to the field of cytogenetics and the application of genetic diagnostic techniques in pathology and haematology. Specifically, the invention relates to nucleic acid probes that can be used in hybridisation techniques for the detection of chromosomal aberrations and other gene rearrangements such as immunoglobulin (Ig) and T cell receptor (TCR) gene rearrangements.

Chromosomal aberrations are a leading cause of genetic disorders or diseases, including congenital disorders and acquired diseases such as malignancies. At the basis of the above malignancies lies the fact that all cells of a malignancy have a common clonal origin. Chromosomal aberrations in malignancies stem from rearrangements, translocations, inversions, insertions, deletions and other mutations of chromosomes, but also losses or gains of whole chromosomes are found in malignancies. In many chromosome aberrations two different chromosomes are involved. In this way, genes, or fragments of genes are removed from the normal physiological context of a particular chromosome and are located to a recipient chromosome, adjacent to non-related genes or fragments of genes (often oncogenes or proto-oncogenes). Such an aberrant genetic combination can be the foundation of a malignancy.

Often, such rearrangements involving two non-aberrant chromosomes happen in a somewhat established pattern. Breaks occur in either of the two chromosomes at a potential breakpoint or breakpoint cluster region resulting in removal of a gene or gene fragment from one chromosome and subsequent translocation to the other chromosome thereby forming a rearranged chromosome where the rearranged fragments are fused in a fusion region.

Detection of chromosome aberrations can be achieved using a wide array of techniques, various of which entail modern biomolecular technology. Traditional techniques such as cytogenetic analyses by conventional chromosome banding techniques are, although highly precise, very labour intensive, require skilled personal and are expensive. Automated karyotyping is useful for some diagnostic applications, such as prenatal diagnosis, but is ineffective in analysing the complex chromosomal aberrations of many malignancies. Furthermore, above techniques require fresh (cultured) cells, which are not always available.

Other, more modern, techniques are Southern blotting or other nucleic acid hybridisation techniques or amplification techniques such as PCR, for the detection of well-defined chromosome aberrations for which suitable nucleic acid probes or primers are available. With these techniques, fresh or frozen cells can be used, and sometimes even samples after formalin fixation as long as the nucleic acid sequences to be hybridised or amplified remain intact and accessible. However, even with above modern technology, several disadvantages can be found that hamper the application of these diagnostic techniques in the rapid screening for chromosomal aberrations related to said malignancies.

Southern blotting lasts 3 to 4 weeks, which is too slow for efficient diagnosis and choice of therapy in malignancies, and allows only 10-15 kb of nucleic acid sequences to be analysed per probe analysis.

PCR, although in essence well-suited for rapid and massive diagnostic testing or even screening, allows only 0.1 to 2 kb of nucleic acid to be analysed per PCR analysis, which greatly hampers rapid screening of vast stretches of chromosomes and breakpoint cluster regions within the chromosomes. An additional disadvantage of PCR is its inherent sensibility to mismatched primers. Small, normal, and physiological alterations which can always be present in the nucleic acid sequence of the gene fragment complementary to the primer hamper the reliable application of PCR and eventually give rise to false-negative results. Especially false-negative results render a PCR-based diagnostic test, albeit very specific, not sensitive enough for reliable diagnosis, and it goes without saying that only a reliable diagnosis of malignancies can contribute to an understanding of the prognosis and the design of an adequate therapy.

Fluorescent *in situ* hybridisation techniques (FISH) are less dependent on the complete matching of nucleic acid sequences to get positive diagnostic results. In general FISH employs probe analyses with large, mainly unspecified, nucleic acid probes that hybridise, however often with varying stringency, with the genes or gene fragments located at both sides of the fusion region in the rearranged chromosome in the malignant cell. Using large probes renders the FISH technique very sensitive. The binding of the colocalizing probes is generally detected either directly or indirectly with fluorochromes and visualised via fluorescence microscopy of a population of cells obtained from the sample to be tested.

However, even the currently used FISH protocols have inherent disadvantages, these mainly relate to the selection of nucleic acid probes employed in the current FISH protocols, which can give false-positive results in the diagnosis of chromosomal aberrations. For example, probes directed against different chromosomes with juxta position of signals in case of translocation create a rather large risk of false-positive results. Hence, the diagnostic tests are, albeit sensitive, not specific enough to employ standard FISH techniques in massive or rapid diagnostic testing, let alone in automated testing or screening.

Thus far, generally large probes, derived of cosmid clones, YAC clones, or other cloned DNA fragments have been used as probes in FISH. The exact position of these probes in relation to the fusion region in the rearranged chromosome is unknown and they are of largely unspecified and varying genomic length (genomic length or distance as expressed as the number of nucleotides or bases (b)) and go without specific selection or modification of these probes beyond the mere labeling of the probes with the necessary reporter molecules i.e. fluorochromes. For designing or selecting probes, little or none guidance is given in the art, beyond mere suggestions as to where to localise putative probe. False-positive results obtained with these probes may stem from aspecific hybridisation with a wide array of (major) repetitive sequences present throughout various chromosomes, or from cross-hybridisation to homologous sequences in the genome, or from overlap of the used probes with the breakpoint cluster region or from the difference in signal intensities as far as originating from size differences of the probes. These causes of false-positive results are frequently not recognized. False-positive results are especially detrimental to rapid diagnosis if rapid or routine screening of patients is needed to detect malignancies or in evaluating treatment protocols. A false-positive result then necessitates cumbersome re-testing of patients, or even unsuspecting clients that have been submitted to routine screening protocols, and can greatly alarm these people. Furthermore, translocations are generally detected with two different probes, one for each of the involved chromosomes, which probes than colocalise during the *in situ* hybridisation in case of a translocation, but show separate signals when no translocation is present (see for example EP 0430402, EP 0500290 A, Tkachuk et al., Science 250, 559-562, 1990; Tkachuk et al., Clinical applications of fluorescence in situ hybridization", Genetic analysis techniques and applications vol. 8, 67-74, 1991). However, in practice 2 to 4% of normal interphase cells tested by FISH will show false-positive results due to the fact that the two probes colocalize by chance. An additional disadvantage of the current FISH protocols is that it is in practice necessary to know both chromosomes that are involved in the translocation as well as the relevant breakpoint regions of both chromosomes to define the nucleic acid probes enabling the detection of the specified translocation, while as yet unknown or ill-defined translocations originating from a well-known gene and an unknown partner gene remain undetected.

LaForgia S et al. (1993, Cancer Research, 53:3118-3124) discloses the mapping of a large number of probes using Southern blotting, PCR amplification, and FISH around the breakpoint of the t(3;8)-(p14.2;q24.1) translocation which is found in familial renal cell carcinoma (RCC).

Dauwerse JD et al. (1990, Cytogenet. Cell Genet. 53:126-128) discloses the mapping of the breakpoint of the pericentric inversion of chromosome 16, which is characteristic for acute nonlymphocytic leukemia (ANLL), using cosmids and nonradioactive in situ hybridization.

EP 0 430 402 A discloses methods for staining chromosomes employing one or more nucleic acid probes.

Tkachuk DC et al. (1991, Genetic Analysis Techniques and Applications, 8(2):67-74) reviews the clinical applications of single- and multicolr FISH in standard karyotyping, prenatal diagnosis, and tumor cytogenetics.

It is an object of the present invention to provide improved nucleic acid probes and methods for detecting a potential breakpoint of a chromosome.

The solution provided by the present application is a distinct and balanced pair of nucleic acid probes, wherein each nucleic acid probe is labeled with at least one different reporter molecule,
wherein each of the nucleic acid probes is capable of hybridizing to a sequence such that the pair of nucleic acid probes would flank a potential breakpoint of a chromosome upon hybridization to the chromosome,
wherein the probes hybridize at a genomic distance of no more than 100kb resulting in colocalization of the reporter molecule signals if no chromosome aberration is present,
wherein each probe of the pair of nucleic acid probes is labeled with reporter molecules resulting in signals of comparable intensity between the probe,
and wherein there is no overlap of the nucleic acid probes with the breakpoint cluster region.

The present invention provides nucleic acid probes that can be used in diagnostic testing for chromosome aberrations which combine a high sensitivity and a high specificity. The probes provided by the invention can hybridise, *in situ* or *in vivo* or *in vitro* with complementary nucleic acid molecules such as (m)RNA or DNA, as e.g. transcribed by or found in (non-aberrant and/or rearranged) chromosomes. The present invention provides for each translocation analysis a distinct and balanced pair of nucleic acid probes. The probes are distinct in that they each hybridise to a different sequence specifically selected and flanking a distinct potential breakpoint in a non-aberrant chromosome. Furthermore, the pair formed by e.g. probe A and probe B is distinct from the pair formed by e.g. probe A and probe X. Furthermore, in above example probes A, B and X constitute three pairs, A-B, B-X and A-X. The probes in said pair are comparable or balanced in that they are designed to be of for example comparable size or genomic length with the final aim of facilitating the generation of signals of comparable intensity. In addition, said probes are comparably labelled with reporter molecules resulting in signals of comparable intensity. In addition, said probes are each labelled with a different fluorochrome, facilitating detection on one spot of different colour when they colocalize when no aberration is detected. Also, said probes are selected to react with a chromosome, at respective complementary hybridisation sites that are located at comparable distances at each side of a breakpoint or breakpoint cluster region of a chromosome. The distinct and balanced pair of nucleic acid probes provided by the invention entails probes that are for example of comparable size or genomic length, each probe of the pair for example being from 1 to 10 kb, or 7 to 15 kb, or 10 to 20 kb, or 15 to 30 kb, or 20 to 40 kb, or 30 to 50 kb, or 40 to 60 kb, or 50 to 70 kb, or 60 to 80 kb, or 70 to 90 kb, or 80 to 100 kb in length. By using such a distinct and balanced pair of probes flanking a breakpoint region and not overlapping the corresponding fusion region, false-positive diagnosis in hybridisation studies is avoided. The invention further provides a distinct and balanced pair of nucleic acid probes, each being labelled with at least one different reporter molecule. Nucleic acid probes can be labelled with chromophores or fluorochromes (e.g. FITC or TRITC) or by introducing a hapten such as biotin and digoxigenin. Fluorochrome labelled probes can be detected directly. Hybridisation with haptenised nucleic acid probes is followed by indirect detection using chromopores, fluorochromes or enzymes such as peroxidase. The invention further provides a distinct and balanced pair of nucleic acid probes which are characterised in that both probes hybridise to a single corresponding nucleic acid molecule or its complementary strand, or hybridise to one (non-aberrant) chromosome, or hybridise to a fragment thereof, possibly comprising the aberration, instead of two probes that hybridise separately to the two chromosomes that are involved in a given translocation, as currently used in hematology and oncology in general (see for example Tkachuk et al., Science 250, 559-562, 1990; Tkachuk et al., Clinical applications of fluorescence in situ hybridization", Genetic analysis techniques and applications vol. 8, 67-74, 1991).

The invention provides a distinct and balanced pair of nucleic acid probes which hybridise to said nucleic acid molecule at a genomic distance of no more than 100 kb, but preferably no more than 50 kb. The invention provides a distinct and balanced pair of nucleic acid probes which hybridise *in situ,* and can i.e. be used in diagnostic tests entailing FISH techniques. Furthermore, the invention provides a distinct and balanced pair of nucleic acid probes which probes each hybridise *in situ* under varying but generally low-stringent conditions to only a few DNA molecules per cell. The nucleic acid probes composed of several DNA fragments are tested either on metaphase spreads or with Southern blotting for hybridisation sensitivity and specificity to select the probe on containing as little major repetitive sequences as possible, to avoid high background staining. The nucleic acid probes are tested in fiber FISH (i.e. hybridisation on extended single DNA fibers immobilised on glass slides), prior to being employed in diagnostic testing, for mapping and checking their relative positions.

The probes are tested for example to avoid using probes hybridising two repetitive sequences. Probes can consist of sets of various oligonucleotides, thereby avoiding repetitive sequences present in a flanking region. Such sets are distinctly labelled, with separate or distinct reporter molecules for each probe (or set of oligonucleotides) that is aimed at the respective flanking region. Such probes can each consist of multiple labelled oligonucleotides, each hybridising to a distinct area in a flanking region. One probe can for example contain from 10 up to 200 of such oligonucleotides, preferably from 50-150, each oligonucleotide for example being 10-20 nucleotides ling. For example, the intron-exon structure of the MLL gene is described in the Br J Haematol 1996;93:966-972. The manuscript also shows that most breakpoints in the MLL gene are located between exon 9 and exon 14. PNA containing probes can be designed in exon 3 to 8 for the "upstream FISH probe" and in exon 15 to 31 for the "downstream FISH probe". Particularly exon 4 and exon 28 are important for probe design, because these two exons are rather large and therefore can contain most of the PNA probes. PNA oligonucleotides can be synthesized for example for their capacity to hybridise with said exon 4 or exon 28 from the 119Q3 target gene and used in one cocktail as probe for one flanking region.

The invention further provides the use of said distinct and balanced pair of probes in diagnostic testing for chromosome aberrations. The pair of probes according to the invention can be used in the detection of nucleic acid comprising the aberration or fragments of the aberration, or in the detection of cells, *in situ* or *in vitro,* comprising the chromosome aberration. The invention thus provides a pair or pairs of distinct and balanced probes which can be used in the detection of disorders or diseases related to chromosomal aberrations, i.e. malignancies, such as haematopoietic malignancies as further explained below. Furthermore, the invention provides a diagnostic kit or assay comprising a pair of nucleic acid probes according to the invention which can be used in the detection of disorders or diseases related to chromosomal aberrations, i.e. malignancies, such as haematopoietic malignancies with such a diagnostic kit or assay provided by the invention it is e.g. possible to monitor the effects of therapy and detect minimal residual disease or detect early relapse of cancer. One can also identify the origin of bone marrow cells following bone marrow transplantation. One can also detect viral sequences, and their localisation in the chromosome, in cells. More in detail the present invention is described while referring to molecular detection of chromosome aberrations in hematopoietic malignancies but is widely applicable for analysis of chromosome aberrations in general.

The development of reliable probes for detection of well-defined or even ill-defined chromosome aberrations in hematological malignancies is described as non-limiting example to illustrate the invention. Such probes can be used for diagnosis and for molecular classification of the involved malignancies. The new probes can be used in diagnostic testing in several types of hematological malignancies with increased sensitivity, specificity, and efficacy of analysis.

Each year world-wide many cases of hematopoietic malignancies are being diagnosed. In the European Union (~375 million inhabitants) this concerns ~98,000 patients per year. The estimated number of patients in the USA (~250 million inhabitants) is ~65,500 per year. The majority of hematological malignancies are of lymphoid origin: acute lymphoblastic leukemias (ALL), chronic lymphocytic leukemias, most malignant lymphomas, and multiple myelomas. The non-Hodgkin's lymphomas (NHL) form the largest group, representing approximately half of all hematopoietic malignancies. Furthermore, European epidemiological studies show that the incidence of NHL is gradually increasing (~5% per year), which indicates that NHL poses a significant public health problem in Europe and most probably throughout the Western world. Although the annual number of patients diagnosed with ALL is smaller than for NHL, ALL has a high prevalence in children, representing the most frequent malignancy in childhood.

Lymphoid malignancies consist of a broad range of ~25 different disease entities, which differ in clinical presentation, prognosis, and treatment protocols. These disease entities have been defined in the recent Revised European American Lymphoid neoplasm (REAL) classification. In this classification the lymphoid malignancies are divided in B-cell malignancies (~90%) and T-cell malignancies (~10%).

The diagnosis and classification of lymphoid malignancies is generally based on cytomorphology and histomorphology, complemented with immunophenotypic information via flow cytometry and/or immunohistochemistry. This immunophenotypic information appears to be valuable for classification of lymphoid malignancies, such as the classification of ALL into pro-B-ALL, common-ALL, pre-B-ALL, and several types of T-ALL. In mature B-cell malignancies with immunoglobulin (Ig) expression the diagnosis can be supported by immunophenotypic clonality assessment via detection of single Ig light chain expression, i.e. the distribution of Igκ and Igλ positive B-cells, which is heavily skewed in case of a B-cell malignancy.

The value of clonality assessment is based on the fact that all cells of a malignancy have a common clonal origin. In lymphoid malignancies this is reflected by the presence of identically (clonally) rearranged Ig and T cell receptor (TCR) genes: clonal Ig and/or TCR gene rearrangements are found in most (90-95%) immature lymphoid malignancies and virtually all (>98%) mature lymphoid malignancies. Therefore molecular clonality analysis of Ig and TCR genes is highly suitable for discrimination between monoclonal (malignant) and polyclonal (reactive) lymphoproliferations. Suspect lymphoproliferations should therefore be subjected to molecular clonality assessment.

During the last decade the knowledge about genetic aberrations in hematopoietic malignancies has considerably increased, especially in acute leukemias and NHL. Currently, well-established chromosome aberrations are found in 35-40% of ALL and in 30-40% of NHL. These chromosome aberrations can be used as alternative or additional markers for molecular clonality assessment. More importantly, these chromosome aberrations appear to be relevant classification markers, which supplement the currently used morphological and immunophenotypic classification systems. It has been clearly demonstrated that several genetic aberrations are associated with a favourable prognosis, whereas others are associated with poor prognosis, such as t(4;11) in pro-B-ALL and t(9;22) in common-ALL. Several treatment protocols have started to use this information for stratification of treatment. Therefore it can be anticipated that rapid and reliable detection of well-defined genetic aberrations will become essential in the diagnosis and management of hematopoietic malignancies.

Several different types of chromosome aberrations have been identified in ALL and NHL. The chromosome aberrations in precursor-B-ALL mainly concern translocations, which result in fusion genes, encoding for fusion proteins with new or modified functions. Examples include the E2A-PBX and BCR-ABL fusion proteins, resulting from t(1;19) and t(9;22), respectively. Another important chromosome region, the 11q23 region with the *MLL* gene, is involved in several types of translocations in acute leukemias. In these 11q23 translocations different partner genes are involved, leading to different fusion proteins. One of them is t(4;11), which is observed in ~70% of infant acute leukemias. Many chromosome aberrations in T-ALL and NHL involve Ig or TCR gene sequences in combination with oncogene sequences. These chromosome aberrations do not give rise to fusion proteins, but result in increased or stabilized expression of the involved oncogene, thereby contributing to uncontrolled growth. They occur at relatively high frequency in particular disease categories, such as t(14;18) with involvement of the *BCL2* gene in ~90% of follicular lymphomas and t (11;14) with involvement of the *BCL1*/Cyclin D1 gene in ~70% of mantle cell lymphomas.

From origin cytogenetic analysis of chromosomes has been the standard technique for detection of chromosome aberrations. This technique needs the presence of cells in metaphase, which generally requires various cell culture systems, dependent on the type of malignancy. The success rate for obtaining reliable karyograms is highly dependent on the type of malignancy and the experience of the laboratory and ranges from less than 50% to over 90%. Furthermore, some chromosome aberrations can not or hardly be detected by cytogenetic analysis such as *TAL1* deletions in T-ALL and t(12;21) in precursor-B-ALL. Therefore in case of well-established chromosome aberrations the labor-intensive and time-consuming classical cytogenetics is now being replaced by molecular techniques. As said, molecular analysis of genetic aberrations can be performed with Southern blotting, polymerase chain reaction (PCR) techniques, and FISH techniques.

Southern blot analysis has long been the most reliable molecular method for detection of well-established chromosome aberrations, but this technique is dependent on the availability of suitable DNA probes, which recognize all relevant breakpoint cluster regions of the involved chromosome aberrations. The latter probably explains why *BCL2* and *BCL1*/CyclinD1 gene aberrations are detectable by Southern blotting in only 75% of follicular lymphomas and in only 50% of mantle cell lymphomas, respectively. Furthermore, Southern blot analysis is time-consuming and requires relatively large amounts of high-quality DNA derived from fresh or frozen cell samples.

Over the last five years, PCR-based techniques have been developed as alternatives for Southern blotting. PCR techniques have the advantage that they are rapid and require minimal amounts of medium-quality DNA, which might even be obtained from formalin-fixed paraffin-embedded tissue samples. Also mRNA can be used after reverse transcription (RT) into cDNA. RT-PCR is especially valuable in case of chromosome aberrations with fusion genes and fusion transcripts, such as frequently seen in precursor-B-ALL and in t(2;5) in anaplastic large cell lymphoma. Despite these obvious advantages, the broad application of PCR techniques for detection of chromosome aberrations in hematopoietic malignancies is hampered by several problems. False-negative PCR results can be obtained if the DNA or mRNA from formalin-fixed paraffin-embedded tissue samples is less optimal than anticipated, or when primers are mismatching. False-positive results might be obtained due to cross-contamination of PCR products between samples from different patients; especially in case of RT-PCR studies of fusion gene transcripts it might be difficult to exclude false-positive results. Finally, routine PCR analysis can only be used to study relatively small fusion regions of chromosome breakpoints (<2 kb). This implies that multiple oligonucleotide primer sets are needed to cover the most important breakpoint and fusion regions, whereas it will be difficult to study large breakpoint or fusion regions (>10 kb). This explains the lower detectability of chromosome aberrations, and thus again the presence of false-negative results, at the DNA level by PCR as compared to Southern blotting.

A major advantage of FISH techniques as compared to cytogenetic analysis, Southern blotting, and PCR analysis is that FISH can be performed on interphase nuclei of all kind of tissue and cell samples and that there is no need for extraction of DNA or mRNA. In FISH techniques generally large DNA probes (>25 kb) are used, which are located around the breakpoint regions of the two chromosomes of the studied chromosome aberration. This implies that FISH probes can scan much larger regions than Southern blot probes or PCR primers. This advantage is especially important for detection of breakpoints outside the traditional breakpoint cluster regions. Furthermore the use of large fluorescently-labeled DNA probes allow direct and rapid visualization of deletions and translocations of the studied gene regions. Application of the latest generation of fluorescent microscopes with multiple fluorochrome filter combinations, CCD camera, and appropriate computer software allow the combined use of multiple FISH probes, which are labeled with different fluorochromes.

The availability of suitable probes is the main limiting factor in using FISH technology for detection of chromosome aberrations. Thus far, generally cosmid clones, YAC clones, or other cloned DNA fragments have been used without specific selection or modification of these probes. For many of these probes the position in the genome is not precisely known; they often even overlap with breakpoint cluster regions, and they often contain repetitive sequences which cause high background staining. Furthermore, translocations are generally detected by use of two different probes, one for each of the involved chromosomes; these two probes are assumed to colocalize in case of a translocation, but show separate signals if no translocation is present. However, in practice 2 to 4% of normal interphase cells will show false-positive results due to the fact that the two signals colocalize by chance.

For routine applicability of FISH techniques or other probe analysis assays or kits for the detection of chromosome aberrations in the diagnosis and classification of hematopoietic malignancies, it is necessary to design distinct and balanced probes.
1. The probes of the invention are selected to form a distinct and balanced pair of nucleic acid probes; size of the probes is each within certain limits of the genomes to be detected (e.g. 1-10, or 10-30, or 20-40, or 30-50, or 40-60 kb), with the final aim that the intensity of the fluorescent signals of the various probes is comparable.
2. In an additional embodiment of the invention the position of the probes constituting the pair is determined precisely, i.e. no overlap with breakpoint cluster regions, the relevant breakpoints are preferably located within 50 kb or preferably even within 25 kb of either probe, and an additional probe pair has to be designed, if two breakpoint regions of a particular chromosome aberration are separated for more than 30-50 kb depending on the exact position of the probes.
3. In a further embodiment the nucleic acid probes do not contain (major) repetitive sequences, and do not cross-hybridise, which results in high background staining. For this reason the nucleic acid probes composed of several fragments can be tested either on metaphase spreads or with Southern blotting for hybridisation sensitivity and specificity.
4. The nucleic acid probes can alternatively, or additionally be tested in fiber FISH prior to being employed in diagnostic testing, for mapping and checking their relative positions.
5. It has additionally been found that detection of chromosome breakpoints becomes easier and more reliable, if two separate probes, labelled with two different fluorochromes, constituting said pair are designed around one of the breakpoint regions of a chromosome aberration. This will lead to colocalization of the signals if no breakpoint is present. However if a breakpoint occurs in the studied breakpoint region, the two differently labeled probes will result in two separate signals.
6. In addition, the design of a third probe (labeled with a third fluorochrome) and thus the design of two additional distinct pairs of probes for the partner gene of the chromosome aberration allows precise identification of the chromosome aberration.

Chromosome aberrations found in malignancies are useful for molecular classification, such as in case of acute leukemias, malignant lymphomas and solid tumors (Table 1). However, several of these aberrations are more important than others, because of their high frequency or because of their prognostic value. For instance, t(14;18) occurs frequently in NHL, whereas t(12;21) is frequently found in childhood precursor-B-ALL. On the other hand, translocations involving the MLL gene in the 11q23 region represent a poor prognostic factor and the presence of 11q23 (MLL gene) aberrations is already in use as an important factor for stratification of treatment in acute leukemias. Also t(9;22) in ALL has a poor prognosis and is used for treatment stratification

The *MLL* (for myeloid-lymphoid leukemia or mixed-lineage leukemia) gene in chromosome region 11q23 is involved in several translocations in both ALL and acute myeloid leukemias (AML). In these translocations the MLL gene, encoding a protein that shows homology to the Drosophila trithorax gene product, is fused to partner genes on different chromosomes. To date at least ten partner genes have been identified. Some of these translocations, like the t (4;11) (q21;q23), t (11;19) (q23;p13) and t (1;11) (p32;q23), predominantly occur in ALL, whereas others, like t (1;11) (q21;q23), t(2;11) (p21;q23), t(6;11) (q27;q23) and t(9;11) (p22;q23) are more often observed in AML. Other types have been reported in ALL as well as AML. Treatment-induced AML with 11q23 aberrations can arise in patients previously treated with topoisomerase II inhibitors. Rearrangements involving the 11q23 region occur very frequently in infant acute leukemias (around 60-70%), and to a much lesser extent in childhood and adult leukemias (each around 5%). *MLL* gene rearrangements, especially the t(4;11), have been shown to be a poor prognostic factor in infant leukemias, resulting in a 3-year overall survival of 5% as compared to 85-90% in cases with germline *MLL* genes.

The large *MLL* gene (>100 kb) consists of 21 exons, encoding over 3900 amino acids. Breakpoints in the *MLL* gene are clustered in a 8.5-9 kb region that encompasses exons 5-11. Because of its relatively small size, this breakpoint region is easily accessible for molecular detection of translocations. By choosing two distinctly-labeled FISH probes in the sequences flanking the breakpoint region, any translocation involving the 11q23 region can be detected on the basis of segregation of the two fluorochrome signals, whereas the two fluorochromes colocalize when no rearrangement in the *MLL* gene has occurred. Furthermore, the use of a third fluorochrome for probes directed against partner genes enables the identification of the precise type of translocation. This two-step approach of FISH analysis guarantees efficient and direct detection of all aberrations involving the 11q23 (*MLL* gene) region in the first step, whereas in the second step the type of 11q23 translocation can be determined.

Chromosome aberrations in lymphoid malignancies often involve Ig or TCR genes. Examples include the three types of translocations (t(8;19), t(2;8), and t(8;22)) that are found in Burkitt's lymphomas, in which the *MYC* gene is coupled to Ig heavy chain (IGH), Ig kappa *(IGK),* or Ig lambda (*IGL*) gene segments, respectively. Another common type of translocation in this category is the t(14;18)(q32;q21) that is observed in ~90% of follicular lymphomas, one of the major NHL types. In this translocation the *BCL2* gene is rearranged to regions within the *IGH* locus within or adjacent to the JH gene segments. The result of this chromosome aberration is the overexpression of the BCL2 protein, which plays a role as survival factor in growth control by inhibiting programmed cell death.

The *BCL2* gene consists of only three exons, but these are scattered over a large area. Of these the last exon encodes a large 3' untranslated region (3' UTR). This 3' UTR is one of the two regions in which many of the t(14;18) breakpoints are clustered and is called "major breakpoint region" (mbr); the other breakpoint region involved in t(14;18) translocations, is located 20-30 kb downstream of the *BCL2* locus and is called the "minor cluster region" (mcr). A third *BCL2* breakpoint area, the vcr (variant cluster region), is located at the 5' side of the *BCL2* locus and is amongst others involved in variant translocations, i.e. t(2;18) and t(18;22), in which *IGK* and *IGL* gene segments are the partner genes.

By choosing a set of FISH probes that are located in the regions upstream of the mbr region and downstream of the mcr region, translocations in these regions can be detected upon segregation of the fluorochrome signals. An additional set of FISH probes is designed for the vcr region, since the distance between the vcr region and the other two breakpoint clusters is far too large (~400 kb) to use the same probes. As a second step in all these approaches, FISH probes in the *IGH, IGK,* and *IGL* genes are used for identification of the exact type of translocation.

Several types of nucleic acid probes can be employed in FISH technology as provided by the invention for detection of chromosome aberrations. Each of these probe types has its own characteristic features and advantages, together constituting a complementary approach, i.e. cosmid, PAC, or YAC derived probes, PCR-based probes, or PNA-based probes.

Clones obtained from cosmid, PAC or YAC libraries constitute large probes that, when labeled with fluorochromes, result in appropriate signals upon hybridization (Gingrich et al., 1996). However, conventionally as the precise position of these probes is unknown, there often is a risk of overlap with the breakpoint cluster region of the involved chromosome aberration, if no further selection or modification of these probes is performed. Furthermore, such large probes often contain repetitive sequences, which cause high background staining. Distinct and balanced pairs of probes comprising cosmid, PAC or YAC probes that are designed to react with the flanking regions upstream and downstream of the breakpoint area on one of the involved chromosomes can therefore be exactly positioned in fiber FISH experiments or by use of Southern blotting using small well-defined inclusion and exclusion probes which are designed around the breakpoint area avoiding said overlap with the breakpoint cluster. The presence of potential repetitive sequences is excluded via Southern blot analysis of genomic DNA.

Probes that are generated by PCR have the additional advantage that they can be positioned exactly, however for this approach sequence information is required at least in the areas for designing the target-specific PCR primers for producing the probes. Once generated the PCR products are checked for the presence of repetitive or cross-hybridizing sequences that hamper specific detection of the flanking regions upstream and downstream of the involved break point cluster. PNA-based probes for FISH technology comprise multiple (e.g. 50-150) distinct PNA oligonucleotides each of which shows a typical size of 5-40, more typically 10-25 nucleotides and which together produce an appropriate signal for detection of chromosome aberrations using FISH technology. PNA probes, having a neutral peptide backbone to which the four deoxynucleotides are coupled are stable nucleic acid fragments that hybridize to complementary nucleic acid sequences with high affinity (Egholm et al., 1993; Corey, 1997). Due to the fact that mismatches strongly influence PNA hybridization, sequence specificity of PNA recognition can be easily achieved, therby rendering PNA probes highly selective probes to be used in FISH technology. PNA probes have now been used in a variety of applications inclusing *in situ* hybridization to highly repetitive contromeric or telomeric sequences (Corey, 1997). Thusfar, only a single PNA oligonucleotide directed against repeated sequences was sufficient for appropriate signal intensities. The design of a balance pair of nucleic acid probes comprising multiple (e.g. 50-150) distinct PNA oligonucleotides directed against target sequences in the flanking regions of a breakpoint cluster provides detection of chromosome aberrations as well as with other nucleic acid probes.

### Applicability of the various types of probes

Each of the before mentioned types of FISH probes has its specific applicability, but together they constitute complementary and partly overlapping strategies.

The *MLL* gene in chromosome region 11q23 is an example of a detectable region that is involved in several translocations in both ALL and AML (Table 1), providing a perfect example of a chromosome aberration for which PCR-based or PNA-based FISH probes can preferably be designed. Because the breakpoint area in the MLL gene is so tightly clustered with ample exons available in the flanking regions upstream and downstream of the breakpoint cluster, sequence-based design and production of distinct and balanced pairs of PCR-based and/or PNA-based FISH probes is very useful in this chromosome region. Design of precisely positioned cosmid or PAC clones could be useful as an alternative or additional strategy.

The *BCL2* gene area is an example that is involved in several chromosome aberrations in malignant lymphomas (Table 1) which contains several breakpoint areas which are located outside the coding sequence at the 5' and 3' sides of the *BCL2* locus and which are lying far apart. The *BCL2* locus therefore exemplifies a gene involved in chromosome aberrations for which distinct and balanced pairs of FISH probes are more difficult to generate via PCR and/or via pooling of PNA oligonucleotides, as less sequence information is available. In such chromosome aberrations distinct and balanced pairs of cosmid, PAC, and/or YAC derived FISH probes can be employed after careful selection and modification of the exact position.

### REFERENCES

1. Gingrich J.C., Boehrer D, Garnes J.A., Johnson W., Wong B., Bermann A., Eveleth G.G., Longlois R.G., Carrano A.V. Construction and characterization of human chromosome 2-specific cosmid, fosmid, and PAC clone libraries. Genomics 1996:32:65-74.
2. Egholm M., Buchard O., Christensen L., Behrens C., Freier S.M., Driver S.A., Berg R.H., Kim S.K., Noreden B., Nielsen P.E. PNA hybridizes to complementary oligonucleotides obeying the Watson-Crick hydrogen-bonding rules. Nature 1993:365:566-568
3. Corey D.R. Peptide nucleic acids: expanding the scope of nucleic acid recognition. Tibtech 1997;15:244-229.
4. Wiegant J., Kalle W., Mullenders L., Brookes S., Hoovers J.M.N., Dauwerse J.G., Van Ommen G.J.B., Raap A.K. High-resolution *in situ* hybridization using DNA halo preparations. Hum.Mol.Genet 1992:5:17-21
5. Young B.D., Saha V. Chromosome Abnormalities in Leukaemia: The 11q23 Paradigm. Cancers Surveys 1996;28:225-245
6. Nilson I., Löchner K., Siegel G., Griel J., Beck J.D., Fey G.H., Marschalek R. Exon/ingron structure of the human ALL-1 (MLL) gene involved in translocations to chromosomal region 11q23 and acute leukaemias. Br J Haematol 1996;93:966-972
7. Taki T., Ida K., Bessho F., Hanada R., Kikuchi A., Yamamoto K., Sako M., Tsuchida M., Seto M., Ueda R., Hayashi Y. Frequency and clinical significance of the MLL gene rearrangements in infant acute leukemia. Leukemia 1996;10:1303-1307
8. Gascoyne R.D., Adomat S.A., Krajewski S., Kajewska M., Horsman A., Tolcher A.W., O'Reilly S.E., Hoskins, Coldman A.J., Reed J.C., Connors J.M. Prognostic significance of Bcl-2 protein expression and Bc12-gene rearrangement in diffuse aggressive Non-Hodgkin's lymphoma. Blood 1997;90:244-251
9. Seto M., Jaeger U., Hockett R.D., Graninger W., Benett S., Goldman P., Korsmeyer S.J. Alternative promoters and exons, somatic mutation and deregulation of the *BCL2-1g* fusion gene in lymphoma. EMBO J 1988;7:123-131
10. Seite P., Leroux D., Hillion J., Monteil M., Berger R., Mathieu-Mahul D., Larsen C.J. Molecular analysis of a variant 18;22 translocation in a case of lymphocytic lymphoma. Genes Chrom Cancer 1993;6:39-44
11. Tashiro S., Takechi M., Asou H., Takauchi K., Kyo T., Dohy H., Kikuchi M., Kamada N., Tsjujimoto Y. Cytogenetic 2;18 and 18;22 translocation in chronic lymphocytic leukemia with juxtaposition of *bcl-2* and immunoglobulin light chain genes. Oncogene 1992;7:573-577
12. Hibshoosh H., Lattes R. Immunohistochemical and molecular genetic approaches to soft tissue tumor diagnosis: a primer. Semin Oncol 1997;24:515-525
13. Zoubek A., Dockhorn-Dworniczak B., Delattere O., Christiansen H., Niggli F., Gatterer-Menz I., Smith T.L., Jürgens H., Gadner H., Kovar H. Does expression of different *EWS* chimeric transcripts define clinically distinct risk groups of Ewing tumor patients? J Clin Oncology 1996;14:1245-1251

**Table 1. Examples of chromosome aberrations in malignancies, that are detectable with a distinct and balanced pair of nucleic acid probes of the invention.**

| Translocation | Involved genes | Primary target gene for FISH probe design | Occurrence per disease category |
|---|---|---|---|
| *Acute leukemias* | | | |
| t(4;11)(q21;Q23) | *MLL-AF4* | | - 70% of infant ALL |
| t(11;19)(q23;p13) | *MLL-ENL* | *MLL* gene | - 5 - 7% of ALL |
| t(6;11) (q27;q23) | *MLL-AF6* | | - 5-6% of AML |
| t(9;11)(p22;q23) | *MLL-AF9* | | |

| *Malignant lymphomas* | | | |
|---|---|---|---|
| t(14;18)(q23;q21) | *BCL2-IGH* | | - 90% of follicular |
| t(2;18)(p12;q21) | *IGK-BCL2* | *BCL2 gene* | NHL |
| t(18;22)(q21;q11) | IGL-BCL2 | | - 25% of immunoblastic |
| | | | NHL |
| | | | - 25% of diffuse large |
| | | | cell centroblastic NHL |
| | | | - 5-10% of B-CLL |

| *Solid tumors* | | | |
|---|---|---|---|
| t(11;22)(q24;q12) | *EWS-FLI1* | | >95% of Ewing sarcoma |
| t(21;22)(q22;q12) | *EWS-ERG* | *EWS* gene | |
| t(7;22)(p22;q12) | *EWS-ETV1* | | |

## Claims

1. A distinct and balanced pair of nucleic acid probes,
wherein each nucleic acid probe is labeled with at least one different reporter molecule,
wherein each of the nucleic acid probes is capable of hybridizing to a sequence such that the pair of nucleic acid probes would flank a potential breakpoint of a chromosome upon hybridization to the chromosome,
wherein the probes hybridize at a genomic distance of no more than 100kb resulting in colocalization of the reporter molecule signals if no chromosome aberration is present,
wherein each probe of the pair of nucleic acid probes is labeled with reporter molecules resulting in signals of comparable intensity between the probe,
and wherein there is no overlap of the nucleic acid probes with the breakpoint cluster region.

2. A pair of nucleic acid probes according to claim 1, wherein each of the nucleic acid probes is made up of multiple oligonucleotides.

3. A pair of nucleic acid probes according to any preceding claim, wherein each nucleic acid probe is labeled directly with at least one reporter molecule.

4. A pair of nucleic acid probes according to any preceding claim, wherein the at least one reporter molecule is chosen from enzymes, chromophores, fluorochromes, and haptens, such as biotin or digoxygemin.

5. A pair of nucleic acid probes according to any preceding claim, wherein the pair of nucleic acid probes hybridizes to a corresponding nucleic acid molecule which is at least a fragment of a chromosome.

6. A pair of nucleic acid probes according to any preceding claim, wherein the pair of nucleic acid probes is suitable for hybridizing *in situ.*

7. Use of a pair of nucleic acid probes according to any of claims 1 to 6 for the in vitro detection of a nucleic acid molecule comprising a chromosome aberration.

8. Use of a pair of nucleic acid probes according to any of claims 1 to 6 for the in vitro detection of cells comprising a chromosome aberration.

9. Use of a pair of nucleic acid probes according to any of claims 1 to 6 for the in vitro detection of a disorder or disease caused by a chromosome aberration.

10. Use of a pair nucleic acid probes according to any of claims 7 to 9, wherein the chromosome aberration is related to a malignancy.

11. Use of a pair of nucleic acid probes according to any of claims 7 to 10, wherein the chromosome aberration is related to a hematopoietic malignancy.

12. A diagnostic kit comprising at least a pair of nucleic acid probes according to any of claims 1 to 6.

13. A diagnostic kit according to claim 12 further comprising at least one additional nucleic acid probe.

14. A process for detecting a chromosome aberration in vitro, **characterized in that** a pair of probes according to any of claims 1 to 6 is hybridized to chromosomes, the colocalization of the probes indicating a normal chromosome and the separation of the probes indicating a chromosome aberration.

15. A process according to claim 14, **characterized in that** the hybridization is an *in situ* hybridization.

16. A process according to claims 14 or 15, **characterized in that** the hybridization is to interphase nuclei.

17. A process according to any of claims 14 to 16, **characterized in that** the detected chromosome aberration causes a disorder or disease.

18. A process according to any of claims 14 to 17, **characterized in that** the chromosome aberration is related to a malignancy.

19. A process according to any of claims 14 to 18, **characterized in that** the chromosome aberration is related to a hematopoietic malignancy.

## Patentansprüche

1. Eindeutiges und abgestimmtes Nukleinsäuresondenpaar,
wobei jede Nukleinsäuresonde mit mindestens einem unterschiedlichen Reportermolekül markiert ist,
wobei jede der Nukleinsäuresonden mit einer Sequenz derart hybridisieren kann, dass das Nukleinsäuresondenpaar einen potentiellen Bruchpunkt auf einem Chromosom bei der Hybridisierung an das Chromosom flankieren würde,
wobei die Sonden in einem genomischen Abstand von nicht mehr als 100 kb hybridisieren, der zu einer Co-Lokalisation der Signale der Reportermoleküle führt, falls keine Chromosomenanomalie vorliegt,
wobei jede Sonde des Nukleinsäuresondenpaars mit Reportermolekülen markiert ist, die zu Signalen vergleichbarer Intensität der beiden Sonden führen,
und wobei es keine Überlappung der Nukleinsäuresonden mit der Bruchpunkt-Cluster-Region gibt.

2. Nukleinsäuresondenpaar nach Anspruch 1, wobei jede der Nukleinsäuresonden aus mehreren Oligonukleotiden aufgebaut ist.

3. Nukleinsäuresondenpaar nach einem der vorhergehenden Ansprüche, wobei jede Nukleinsäuresonde mit mindestens ei nem Reportermolekül direkt markiert ist.

4. Nukleinsäuresondenpaar gemäß einem der vorhergehenden Ansprüche, wobei das mindestens eine Reportermolekül aus Enzymen, Chromophoren, Fluorochromen und Haptenen, wie zum Beispiel Biotin oder Digoxigenin, gewählt ist.

5. Nukleinsäuresondenpaar nach einem der vorhergehenden Ansprüche, wobei das Nukleinsäuresondenpaar an ein korrespondierendes Nukleinsäuremolekül hybridisiert, das zumindest ein Fragment eines Chromosoms darstellt.

6. Nukleinsäuresondenpaar nach einem der vorhergehenden Ansprüche, wobei das Nukleinsäuresondenpaar zum Hybridisieren *in situ* geeignet ist.

7. Verwendung eines Nukleinsäuresondenpaars nach einem der Ansprüche 1 bis 6 zum *in-vitro*-Nachweis eines Nukleinsäuremoleküls, das eine Chromosomenanomalie umfasst.

8. Verwendung eines Nukleinsäuresondenpaars nach einem der Ansprüche 1 bis 6 zum *in-vitro-*Nachweis von Zellen, die eine Chromosomenanomalie umfassen.

9. Verwendung eines Nukleinsäuresondenpaars nach einem der Ansprüche 1 bis 6 zum *in-vitro*-Nachweis einer durch eine Chromosomenanomalie verursachten Beeinträchtigung oder Krankheit.

10. Verwendung eines Nukleinsäuresondenpaars nach einem der Ansprüche 7 bis 9,
wobei die Chromosomenanomalie mit einer Malignität in Beziehung steht.

11. Verwendung eines Nukleinsäuresondenpaars nach einem der Ansprüche 7 bis 10, wobei die Chromosomenanomalie mit einer hämatopoetischen Malignität in Beziehung steht.

12. Diagnostischer Kit, enthaltend mindestens ein Nukleinsäuresondenpaar nach einem der Ansprüche 1 bis 6.

13. Diagnostischer Kit nach Anspruch 12, der weiterhin mindestens eine zusätzliche Nukleinsäuresonde enthält.

14. Verfahren zum Nachweisen einer Chromosomenanomalie *in vitro,* **dadurch gekennzeichnet, dass** ein Sondenpaar nach einem der Ansprüche 1 bis 6 an Chromosomen hybridisiert wird, wobei die Co-Lokalisation der Sonden ein normales Chromosom anzeigt und die Trennung der Sonden eine Chromosomenanomalie anzeigt.

15. Verfahren nach Anspruch 14, **dadurch gekennzeichnet, dass** es sich bei der Hybridisierung um eine *in-situ*-Hybridisierung handelt.

16. Verfahren nach den Ansprüchen 14 oder 15, **dadurch gekennzeichnet, dass** es sich um eine Hybridisierung an Interphasekerne handelt.

17. Verfahren nach einem der Ansprüche 14 bis 16, **dadurch gekennzeichnet, dass** die nachgewiesene Chromosomenanomalie eine Beeinträchtigung oder Krankheit verursacht.

18. Verfahren nach einem der Ansprüche 14 bis 17, **dadurch gekennzeichnet, dass** die Chromosomenanomalie mit einer Malignität in Beziehung steht.

19. Verfahren nach einem der Ansprüche 14 bis 18, **dadurch gekennzeichnet, dass** die Chromosomenanomalie mit einer hämatopoetischen Malignität in Beziehung steht.

## Revendications

1. Paire distincte et équilibrée de sondes d'acide nucléique,
dans laquelle chaque sonde d'acide nucléique est marquée avec au moins une molécule rapporteuse différente,
dans laquelle chacune des sondes d'acide nucléique est capable de s'hybrider à une séquence de sorte que la paire de sondes d'acide nucléique flanque un point de rupture potentiel d'un chromosome lors de l'hybridation au chromosome,
dans laquelle les sondes s'hybrident à une distance génomique non supérieure à 100 kb, conduisant à une colocalisation des signaux des molécules rapporteuses si une anomalie chromosomique n'est pas présente,
dans laquelle chaque sonde de la paire de sondes d'acide nucléique est marquée avec des molécules rapporteuses conduisant à des signaux d'intensité comparable entre les sondes,
et dans laquelle il n'y a pas de chevauchement des sondes d'acide nucléique avec la région d'amas du point de rupture.

2. Paire de sondes d'acide nucléique selon la revendication 1, dans laquelle chacune des sondes d'acide nucléique est composée de multiples oligonucléotides.

3. Paire de sondes d'acide nucléique selon l'une quelconque des revendications précédentes, dans laquelle chaque sonde d'acide nucléique est marquée directement avec au moins une molécule rapporteuse.

4. Paire de sondes d'acide nucléique selon l'une quelconque des revendications précédentes, dans laquelle ladite au moins une molécule rapporteuse est choisie parmi les enzymes, les chromophores, les fluorochromes et les haptènes, tels que la biotine ou la digoxygénine.

5. Paire de sondes d'acide nucléique selon l'une quelconque des revendications précédentes, dans laquelle la paire de sondes d'acide nucléique s'hybride à une molécule d'acide nucléique correspondante qui est au moins un fragment d'un chromosome.

6. Paire de sondes d'acide nucléique selon l'une quelconque des revendications précédentes, dans laquelle la paire de sondes d'acide nucléique convient à l'hybridation *in situ.*

7. Utilisation d'une paire de sondes d'acide nucléique selon l'une quelconque des revendications 1 à 6, pour la détection *in vitro* d'une molécule d'acide nucléique comprenant une anomalie chromosomique.

8. Utilisation d'une paire de sondes d'acide nucléique selon l'une quelconque des revendications 1 à 6, pour la détection *in vitro* de cellules comprenant une anomalie chromosomique.

9. Utilisation d'une paire de sondes d'acide nucléique selon l'une quelconque des revendications 1 à 6, pour la détection *in vitro* d'un trouble ou d'une maladie provoqués par une anomalie chromosomique.

10. Utilisation d'une paire de sondes d'acide nucléique selon l'une quelconque des revendications 7 à 9, dans laquelle l'anomalie chromosomique est liée à une malignité.

11. Utilisation d'une paire de sondes d'acide nucléique selon l'une quelconque des revendications 7 à 10, dans laquelle l'anomalie chromosomique est liée à une malignité hématopoïétique.

12. Kit de diagnostic comprenant au moins une paire de sondes d'acide nucléique selon l'une quelconque des revendications 1 à 6.

13. Kit de diagnostic selon la revendication 12, comprenant au moins une sonde d'acide nucléique supplémentaire.

14. Procédé pour détecter une anomalie chromosomique *in vitro,* **caractérisé en ce qu'**une paire de sondes selon l'une quelconque des revendications 1 à 6 est hybridée à des chromosomes, la colocalisation des sondes indiquant un chromosome normal et la séparation des sondes indiquant une anomalie chromosomique.

15. Procédé selon la revendication 14, **caractérisé en ce que** l'hybridation est une hybridation *in situ.*

16. Procédé selon les revendications 14 ou 15, **caractérisé en ce que** l'hybridation est à des noyaux interphasiques.

17. Procédé selon l'une quelconque des revendications 14 à 16, **caractérisé en ce que** l'anomalie chromosomique détectée provoque un trouble ou une maladie.

18. Procédé selon l'une quelconque des revendications 14 à 17, **caractérisé en ce que** l'anomalie chromosomique est liée à une malignité.

19. Procédé selon l'une quelconque des revendications 14 à 18, **caractérisé en ce que** l'anomalie chromosomique est liée à une malignité hématopoïétique.
